Europäisches Patentamt

European Patent Office

Office européen des brevets

(19)

(11) Veröffentlichungsnummer: **0 136 549**
**A1**

(12) ## EUROPÄISCHE PATENTANMELDUNG

(21) Anmeldenummer: 84110332.8

(22) Anmeldetag: 30.08.84

(51) Int. Cl.⁴: **C 07 C 19/02**
C 07 C 17/16, C 07 C 17/22

(30) Priorität: 07.09.83 DE 3332253

(43) Veröffentlichungstag der Anmeldung:
10.04.85 Patentblatt 85/15

(84) Benannte Vertragsstaaten:
BE DE FR IT SE

(71) Anmelder: Henkel Kommanditgesellschaft auf Aktien
Postfach 1100 Henkelstrasse 67
D-4000 Düsseldorf-Holthausen(DE)

(72) Erfinder: Rähse, Wilfried, Dr.
Holhagenstrasse 44
D-4000 Düsseldorf 13(DE)

(72) Erfinder: Wüst, Willi, Dr.
Fasanenring 32
D-4030 Ratingen(DE)

(72) Erfinder: Kühne, Norbert
Dürerstrasse 63
D-5657 Haan 1(DE)

(54) Verfahren zur Herstellung von Alkylchloriden aus Alkohol-Ethergemischen, die bei der Celluloseveretherung entstehen.

(57) Beschrieben wird ein Verfahren zur Spaltung von Dimethylether und/oder Diethylether, der bei der Celluloseveretherung anfällt, in die entsprechenden Alkylhalogenide. Das Verfahren arbeitet mit einem Aluminium-Zinkchlorid-Katalysator und kann so geführt werden, daß die Dialkylether enthaltenden Gemische rückstandsfrei in nützliche Produkte überführt werden können.

EP 0 136 549 A1

Croydon Printing Company Ltd

Henkelstraße 67

4ooo Düsseldorf, den 5.9.1983

0136549

HENKEL KGaA
ZR-FE/Patente

Dr.Wik/Po

P a t e n t a n m e l d u n g

D 6701 EP

Verfahren zur Herstellung von Alkylchloriden aus Alkohol-
Ethergemischen, die bei der Celluloseveretherung entstehen.

Die Erfindung betrifft ein Verfahren, das es erlaubt, Nebenprodukte, die beim Verethern von Cellulose mit Methylchlorid und/oder Ethylchlorid entstehen, praktisch vollständig in wertvolle Ausgangsstoffe zu überführen.

Bei der Veretherung von Cellulose mit Alkylhalogeniden in Gegenwart von Natronlauge oder anderer Alkalien entstehen als Nebenprodukte Alkohole und Dialkylether. Diese werden im allgemeinen mit dem überschüssigen Alkylierungsmittel aus den Reaktionsgemischen entfernt oder verbleiben im Suspensionsmittel, aus dem sie durch Destillation abgetrennt werden können.

Um die Wirtschaftlichkeit der Veretherungsverfahren zu erhöhen, wurde bereits in der US-Patentschrift 2 084 710 vorgeschlagen, die Alkoholethergemische mit Salzsäure zu behandeln und sie so wieder in wertvolle Ausgangsstoffe zu überführen. Nach der Lehre dieser Patentschrift werden dazu die Alkoholethergemische unter Druck bei Temperaturen zwischen 80 und 240$^{\circ}$ mit wäßriger Salzsäure behandelt, wobei als Katalysatoren gelöste Schwermetallchloride, insbesondere Zinkchlorid, Antimontrichlorid, Zinnchlorid oder Eisenchlorid zugegen sind. Wenngleich hier der Recyclinggedanke bereits verwirklicht ist, so ist doch die Problemlösung unter heutigen Gesichtspunkten unbefriedigend.

...

So sammeln sich in den verwendeten Lösungen Nebenprodukte an, was insbesondere bei der Herstellung von Mischethern gilt, bei denen Alkylenoxide und ihre Folgeprodukte in den Alkoholethergemischen zugegen sind. Schwierig ist bei diesem Verfahren das Recycling des Katalysators, da die Zinkchloridlösung nicht nur verschmutzt, sondern auch verdünnt wird. Die Katalysatorlösungen müssen deshalb öfters gewechselt werden. Aufgrund ihres hohen Schwermetallgehalts ist es jedoch aufwendig, sie gefahrlos zu beseitigen. Nachteilig ist weiterhin, daß unter Druck gearbeitet werden muß.

Unabhängig vom Problem der Wiederaufarbeitung von Alkoholethergemischen, die bei der Celluloseetherherstellung entstehen, ist bekannt, daß man Methanol oder auch Dimethylether mit Chlorwasserstoffgas in der Gasphase an festen Katalysatoren in Methylchlorid überführen kann. So wurde von E.B.Svetlanov und R.M.Flid in Zh.Fiz.Khim. 40 (12), 3055-9 (1966) ein Katalysator auf Basis Zinkchlorid/Aluminiumoxid mit einem Zinkchloridüberschuß beschrieben. Trotz genereller Eignung weisen die beschriebenen Katalysatoren noch nicht die nötige Aktivität und Standzeit auf, die für ein großtechnisches Verfahren zu fordern sind.

Es bestand somit ein Bedarf nach einem Verfahren, daß es erlaubt, die Nebenprodukte bei der Alkylcelluloseherstellung in einfacher Weise in technisch brauchbare Produkte zu überführen.

Aufgabe der Erfindung ist es somit, ein Verfahren bereitzustellen, daß die Überführung von Alkoholethergemischen aus der Celluloseveretherung in Alkylchloride ermöglicht, ohne daß Schwermetall-Lösungen entstehen bzw. ohne Emission anderer schädlicher Substanzen. Eine weitere Aufgabe der Erfindung ist es, hierzu einen Katalysator hoher Standzeit und Aktivität zur Verfügung zu stellen. Schließlich ist es Aufgabe der Erfindung, ein Verfahren

. . .

zu schaffen, daß die Herstellung besonders reiner Alkylhalogenide aus den Alkoholethergemischen der Celluloseetherherstellung auch dann erlaubt, wenn Alkylenoxide
oder deren Folgeprodukte zugegen sind.

Gegenstand der Erfindung ist somit ein Verfahren zur Aufarbeitung unerwünschter Reaktionsnebenprodukte im Rahmen
der Celluloseetherherstellung unter Gewinnung von Methylchlorid und/oder Ethylchlorid durch katalytische Umsetzung von Gemischen, die Dimethylether, Methanol, Methylchlorid und/oder die entsprechenden Ethylverbindungen
enthalten mit Chlorwasserstoff, dadurch gekennzeichnet,
daß die Umsetzung der Gemische an einem Aluminium-Zinkchlorid-Katalysator mit einem Zinkchloridanteil von 5 bis
45 Gew.-% und bis zu 30 Gew.-% weiteren Promotoren (bezogen auf Gesamtkatalysator) in der Gasphase bei Temperaturen zwischen 130 bis 300$^\circ$C und Drucken bis zu 10 bar
ohne bzw. mit nur geringem Überschuß an Chlorwasserstoff
erfolgt und die anfallenden Alkylchloride durch Wäsche
mit Salzsäure gereinigt werden, worauf die Salzsäure
zur Chlorwasserstofferzeugung zurückgeführt wird.

Das erfindungsgemäße Verfahren geht somit von der Verwendung von Alkoholethergemischen aus, die bei der Alkylcelluloseherstellung durch Kondensation aus der Gasphase
oder durch Destillation aus dem Suspensionsmittel abgetrennt werden können. Ein aus der Gasphase kondensiertes Alkoholethergemisch ist im Falle der Methylcelluloseherstellung beispielsweise folgendermaßen zusammengesetzt:

- 20    bis 70 Gew.-% unumgesetztes Methylchlorid
- 30    bis 80 Gew.-% Dimethylether
- 0,5 bis 20 Gew.-% Methanol.

Im Falle der Herstellung von Ethylcellulose entstehen die

...

entsprechenden Ethylverbindungen, also Ethanol und Diethylether. Gemische, sowie Methylethylether entstehen, wenn Ethylchlorid und Methylchlorid gleichzeitig zur Veretherung eingesetzt werden. Werden als weitere Veretherungsmittel Alkylenoxide mit 2 bis 4 Kohlenstoffatomen eingesetzt, so können die Alkoholethergemische zusätzliche Restmengen dieser Alkylenoxide bis zu 5 Gew.-% sowie deren Ringöffnungsprodukte, insbesondere Glykole, enthalten.

In einer bevorzugten Ausführungsform betrifft die Erfindung ein Verfahren zur Umwandlung der Alkoholethergemische in reine Alkylchloride mit Wasser als einziges Nebenprodukt. Hierzu werden die folgenden Verfahrensstufen vorgeschlagen:

1. Stufe
Destillative Abtrennung der Alkylenoxide

2. Stufe
Katalytische Umwandlung der Alkoholethergemische.

3. Stufe
Reinigung der Alkylchloride durch Abtrennen des überschüssigen Chlorwasserstoffs und der Verunreinigungen mit Salzsäure als Waschflüssigkeit, wobei die Salzsäure in den Prozeß zurückgeführt wird.

4. Stufe
Entwässern von Salzsäure zu Chlorwasserstoff mittels der extraktiven Destillation unter Zugabe von Schwefelsäure und anschließender thermischer Aufkonzentrierung der Schwefelsäure.

...

Die zweite Reaktionsstufe des erfindungsgemäßen Verfahrens umfaßt die katalytische Umwandlung der Alkoholethergemische (Dimethylether und/oder Diethylether enthaltenden Gemische) mit Chlorwasserstoff in der Gasphase an einem festen Katalysator. Als Katalysator ist Aluminiumoxid geeignet, welches 5 bis 45 Gew.-% Zinkchlorid sowie bis zu 30 Gew.-% weiterer Promotoren enthält.

Als weitere Promotoren sind in einer ersten Ausführungsform Alkalimetallchloride geeignet. Diese werden als Alkalihydroxide auf den Aluminiumoxidträgern aufgebracht und bilden sich im feuchten Chlorwasserstoff-Strom. Es können 0,01 bis 5 Gew.-% Alkalimetallchloride eingesetzt werden, vorzugsweise 0,5 - 2 Gew.-%. In einer weiteren Ausführungsform dienen als Promotoren Chloride des Cadmiums, des Eisens oder des Magnesiums. Diese können in Mengen bis zu 30 Gew.-%, bezogen auf Katalysator, verwendet werden.    Es gilt jedoch hier als Regel, daß die Menge dieser Chloride die Menge des Zinkchlorids nicht übersteigen darf. So werden vorzugsweise nur bis zu 75 % des Zinkchlorids durch Cadmium-, Eisen- oder Magnesiumchloride ersetzt. Besonders geeignet sind Katalysatoren, bei denen nur 1 bis 10 Gew.-% der Zinkchloridmengen durch Chloride des Cadmiums, Eisens oder Magnesiums ersetzt worden sind.

Bei dem als Träger verwendeten Aluminiumoxid wird vorzugsweise γ-Aluminiumoxid eingesetzt. Es ist besonders bevorzugt, die im Handel erhältlichen granulierten Formen einzusetzen. Besonders aktive Katalysatoren, welche bei langen Standzeiten nicht zur Abscheidung von Kohlenstoff neigen, werden erhalten, wenn als Träger ein bei 1100°C stoßaktiviertes Aluminiumoxid in Granulatform eingesetzt wird.

. . .

Zur Herstellung des Katalysators wird das granulierte Aluminiumoxid, das vorzugsweise eine Korngröße von 3 bis 5 mm besitzt, eine Stunde bei 800°C geglüht. Nach Abkühlen wird mit einer Zinkchloridlösung, die gewünschtenfalls die weiteren Promotoren enthält, getränkt, zur gleichmäßigen Verteilung gerührt und anschließend das Wasser bei 120 ° durch Trocknen entfernt. Die Katalysatoren werden vor Reaktionsbeginn im Chlorwasserstoffstrom bei Reaktionstemperatur aktiviert.

An den so hergestellten Katalysatoren kann die Umsetzung bei Temperatur zwischen 130°C und 300°C durchgeführt werden. Bevorzugt sind Temperaturen zwischen 150 und $230^{\circ}$, insbesondere zwischen 170 und $210^{\circ}$C. Weiterhin kann die Reaktion bei Unterdruck, Normaldruck oder auch bei Überdruck durchgeführt werden. So ist im weitesten Sinne eine Umsetzung bei Drucken von 0,1 bis 10 bar möglich; bevorzugt ist jedoch das Arbeiten bei 1 bis 1,75 bar. Die Katalysatorbelastung, ohne Inertanteile gerechnet, kann 0,1 bis 6 normal-$m^3$ Reaktionsgas pro Liter Katalysator und Stunde betragen. Vorzugsweise beträgt die Katalysatorbelastung 0,8 bis 2,5 normal-$m^3$ pro Liter und Stunde. Führt man die Reaktion bei Temperaturen unterhalb von 230°C oder sogar unterhalb von 200°C durch, so ist es bevorzugt, Chlorwasserstoff im Überschuß einzusetzen. Der Überschuß an Chlorwasserstoff beträgt dabei bis zu 50 mol-%, vorzugsweise 5 bis 30 mol-%. Selbstverständlich kann auch bei Temperaturen über 230° mit Chlorwasserstoffüberschuß gearbeitet werden.

...

In der dritten Stufe des erfindungsgemäßen Verfahrens werden die entstandenen Alkylchloride gereinigt. Es ist hierzu bevorzugt, sie in einer Kolonne im Gegenstrom mit Wasser oder Salzsäure in Kontakt zu bringen. Dabei beträgt die Temperatur je nach Druck 20 bis 130°C, vorzugsweise 70 bis 110°C. Nach einer weiteren Ausführungsform des erfindungsgemäßen Verfahrens wird nach der Reaktionsstufe das Reaktionsgas auf Temperaturen zwischen 20 und 100°C, vorzugsweise zwischen 40 bis 80° abgekühlt. Dabei kondensiert die aus dem Reaktionswasser und dem Chlorwasserstoffüberschuß entstandene Salzsäure und löst Verunreinigungen der Alkylchloride mit. Gewünschtenfalls kann durch Zugabe weiteren Wassers ein darüber hinaus vorhandener Überschuß an gasförmigem Chlorwasserstoff von den Alkylchloriden abgetrennt werden.

Die nach dieser Stufe erhaltenen Alkylchloride sind weitgehend rein und können ohne weitere Aufarbeitung wieder zur Veretherung von Cellulose eingesetzt werden.

Um das Verfahren völlig rückstandsfrei durchzuführen, werden schließlich in der 4. Stufe die geringe Menge an rezirkulierter Salzsäure mit konzentrierter Salzsäure vermischt und mittels Schwefelsäure zu Chlorwasserstoff entwässert. Die dann verdünnte Schwefelsäure kann durch Destillation aufkonzentriert werden. Bei dieser Verfahrensweise entsteht als einziges Nebenprodukt des erfindungsgemäßen Verfahrens Wasser.

Das erfindungsgemäße Verfahren ist damit besonders umweltfreundlich, da es keine schädlichen Nebenprodukte liefert. Weiterhin zeichnet es sich durch die besonders

. . .

Patentanmeldung    D 6701 EP

HENKEL KGaA
ZR-FE/Patente

0136549

- 8 -

bei kontinuierlicher Betriebsweise wichtige hohe Standzeit und hohe Aktivität der Katalysatoren aus. Die hohe
Standzeit ist insbesondere eine Folge der geringen Neigung zu Kohlenstoffabscheidungen, die ansonsten insbesondere bei den bekannten Aluminiumoxid-Katalysatoren
auftreten können.

B e i s p i e l e

A) Katalysatorherstellung

700 g eines Aluminiumoxidgranulats (bei 1 100 ° Stoß aktiviert) mit einer Korngröße von 3 - 5 mm wurden 1 Stunde
bei 800°C geglüht. Nach Abkühlen auf Raumtemperatur wurden
600 g einer 50 Gew.-%igen Zinkchloridlösung zugegeben.
Nach 30minütigem Durchmengen wurde das Wasser bei 120°C
entfernt. Zur Aktivierung wurde der Katalysator bei
200°C 30 Minuten im Chlorwasserstoffstrom belassen.

B) Umwandlung von Dimethylether und Methanol in Methylchlorid.

Gearbeitet wurde in einem säulenförmigen Festbettreaktor
mit der Länge 3,00 m und 1,3 kg Katalysatorfüllung. Der
Druck, gemessen vor der Schüttung, betrug 1,2 bar. Ausgangsstoffe (Reaktionsteilnehmer), Katalysatorbelastungen
(ohne Berücksichtigung von Inertstoffen wie Wasser) sowie
die erzielten Umsätze sind in der folgenden Tabelle angegeben.

Tabelle: Beispiele 1-6; Versuchsergebnisse am $ZnCl_2-Al_2O_3$-Kontakt

|  | Beispiel-Nr. | | | | | |
|---|---|---|---|---|---|---|
|  | 1 | 2 | 3 | 4 | 5 | 6 |
| Katalysatorbelastung $[normal-m^3/l\ Kat.h]$ | 1 | 1 | 1 | 1,2 | 1 | 1 |
| HCl-Überschuß | 15 % | 10 % | 15 % | 15 % | 15 % | 15 % |
| Temperatur | 215°C | 186°C | 186°C | 217°C | 218°C | 218°C |
| Reaktionsteilnehmer | 100 % DME/HCl | 100 % MeOH/ HCl | 90 % MeOH, 10 % $H_2O$/ HCl | 66,6 % DME, 33,3 % MeOH/ HCl | 66,6 % DME, 31,6 % MeOH, 1,7 % $H_2O$/HCl | 66,6 % DME, 31,5 % MeOH,1,7 % $H_2O$, 0,2 % PO/HCl |
| Umsatz: $U_{DME}$ | 99,8 % |  |  | 98,3 % | 98,5 % | 98,5 % |
| $U_{MeOH}$ |  | 98,8 % | >99 % | ~100 % | ~100 % | ~100 % |

DME: Dimethylether

MeOH: Methanol

HCl: Chlorwasserstoff

PO: Propylenoxid

Zusammensetzung in Gew %

Patentanmeldung   D 6701 EP   - 10 -

HENKEL KGaA
7R-FF/Patente

0136549

Patentansprüche

1. Verfahren zur Aufarbeitung unerwünschter Reaktionsnebenprodukte im Rahmen der Celluloseetherherstellung
unter Gewinnung von Methylchlorid und/oder Ethylchlorid durch katalytische Umsetzung von Gemischen, die
Dimethylether, Methanol, Methylchlorid und/oder die
entsprechenden Ethylverbindungen enthalten mit Chlorwasserstoff, dadurch gekennzeichnet, daß die Umsetzung
der Gemische an einem Aluminium-Zinkchlorid-Katalysator mit einem Zinkchloridanteil von 5 bis 45 Gew.-%
und bis zu 30 Gew.-% weiteren Promotoren (bezogen auf
Gesamtkatalysator) in der Gasphase bei Temperaturen
zwischen 130 bis 300°C und Drucken bis zu 10 bar ohne
bzw. mit nur geringem Chlorwasserstoffüberschuß durchgeführt wird und die anfallenden Alkylchloride durch
Wäsche mit Salzsäure gereinigt werden, worauf die
Salzsäure in das Verfahren zur Chlorwasserstofferzeugung zurückgeführt wird.

2. Verfahren nach Anspruch 1, dadurch gekennzeichnet, daß
als weitere Promotoren bis zu 5 Gew.-% Alkalimetallchloride, welche als Oxide aufgebracht worden sind und/
oder bis zu 30 Gew.-% Chloride des Cadmiums, Eisens
und/oder Magnesiums eingesetzt werden.

3. Verfahren nach den Ansprüchen 1 und 2, dadurch gekennzeichnet, daß als Aluminiumoxid das Granulat eines
bei 1100°C stoßaktiviertem Aluminiumoxids eingesetzt
wird.

4. Verfahren nach den Ansprüchen 1 bis 3, dadurch gekennzeichnet, daß der Druck im Festbettreaktor zwischen 1
und 1,75 bar liegt.                                    ...

- 12 -

5. Verfahren nach den Ansprüchen 1 bis 4, dadurch gekennzeichnet, daß bei einer Katalysatorbelastung von 0,1 bis 6 normal-m$^3$ Reaktionsgas pro Liter Katalysator und Stunde, vorzugsweise zwischen 0,8 bis 2,5 normal-m$^3$ pro Liter Katalysator und Stunde gearbeitet wird.

6. Verfahren nach den Ansprüchen 1 bis 5, dadurch gekennzeichnet, daß bei Temperaturen von 150 bis 230$^o$C, vorzugsweise von 170 bis 210$^o$C, umgesetzt wird.

7. Verfahren nach den Ansprüchen 1 bis 6, dadurch gekennzeichnet, daß die Dimethylether und/oder Diethylether enthaltenden Gemische als weitere Bestandteile Methanol und/oder Ethanol und gegebenenfalls Ethylenoxid, Propylenoxid, 1,2-Butenoxid und/oder deren Hydrolyse Produkte enthalten.

8. Verfahren nach den Ansprüchen 1 bis 7, dadurch gekennzeichnet, daß Chlorwasserstoffgas im Überschuß von 0 bis zu 50 mol-%, vorzugsweise im Überschuß von 5 - 30 mol-%, bezogen auf die stöchiometrisch benötigte Menge, eingesetzt wird.

9. Verfahren nach den Ansprüchen 1 bis 8, dadurch gekennzeichnet, daß die Salzsäure zur Reinigung der Alkylchloride aus überschüssigem HCl und Reaktionswasser bei der Umsetzung selbst entsteht und nach destillativer Abtrennung von Nebenprodukten, mit Schwefelsäure entwässert und in das Verfahren zurückgeführt wird.

## EUROPÄISCHER RECHERCHENBERICHT

| | EINSCHLÄGIGE DOKUMENTE | | EP 84110332.8 |

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | <u>DE - A1 - 3 110 165</u> (HOECHST)<br><br>   * Beispiel *<br><br>-- | 1,4,6,<br>9 | C 07 C 19/02<br><br>C 07 C 17/16<br><br>C 07 C 17/22 |
| A | <u>JP - A - 56-127 324</u> (SHINETSU<br><u>KAGAKU KOGYO K.K.</u>)<br><br>   * Anspruch 1; Beispiel 4;<br>     Seite 3(185), rechte Spalte,<br>     Zeilen 26-30 *<br><br>-- | 1,4,6 | |
| A | <u>JP - A - 52-5 702</u> (CENTRAL GLASS<br>                    K.K.)<br><br>   * Anspruch; Seite 2(6), linke<br>     Spalte, Zeilen 17-20,<br>     rechte Spalte, Zeilen 1,2;<br>     Ausführungsbeispiel 1 *<br><br>-- | 1,4,6,<br>8 | |
| | | | **RECHERCHIERTE SACHGEBIETE (Int. Cl.4)** |
| D,A | RUSSIAN JOURNAL OF PHYSICAL<br>CHEMISTRY, Band 40, Nr. 12, 1966<br><br>E.B. SVETLANOV et al. "Catalytic<br>interaction of hydrogen chloride<br>with methanol. II. Kinetics of<br>the dehydration of methanol and<br>hydrochlorination of methyl ether<br>on catalyst for the vapor-phase<br>synthesis of methyl chloride"<br>Seiten 1638-1641<br><br>   * Gesamt *<br><br>-- | 1,4,6 | C 07 C 19/00<br><br>C 07 C 17/00 |
| D,A | <u>US - A - 2 084 710</u> (SPURLIN)<br><br>   * Ansprüche *<br><br>---- | 1 | |

Der vorliegende Recherchenbericht wurde für alle Patentansprüche erstellt.

| Recherchenort<br>WIEN | Abschlußdatum der Recherche<br>23-11-1984 | Prüfer<br>KÖRBER |
|---|---|---|